(19) Europäisches Patentamt
European Patent Office
Office européen des brevets

(11) **EP 3 041 518 B1**

(12) **EUROPÄISCHE PATENTSCHRIFT**

(45) Veröffentlichungstag und Bekanntmachung des Hinweises auf die Patenterteilung:
**22.11.2017 Patentblatt 2017/47**

(21) Anmeldenummer: **14758976.6**

(22) Anmeldetag: **05.09.2014**

(51) Int Cl.:
*A61L 2/14* (2006.01)    *A61L 2/22* (2006.01)

(86) Internationale Anmeldenummer:
**PCT/EP2014/068919**

(87) Internationale Veröffentlichungsnummer:
**WO 2015/032888 (12.03.2015 Gazette 2015/10)**

(54) **HANDDESINFEKTIONSVORRICHTUNG MIT PLASMA- UND AEROSOLGENERATOR**

HAND DISINFECTION DEVICE HAVING A PLASMA AND AEROSOL GENERATOR

DISPOSITIF DE DÉSINFECTION DES MAINS POURVU D'UN GÉNÉRATEUR DE PLASMA ET D'AÉROSOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.09.2013 DE 102013109777**

(43) Veröffentlichungstag der Anmeldung:
**13.07.2016 Patentblatt 2016/28**

(73) Patentinhaber:
• **Krömker, Wilfried**
  **31675 Bückeburg (DE)**
• **Leibniz-Institut für Plasmaforschung und Technologie e.V.**
  **17489 Greifswald (DE)**

(72) Erfinder:
• **WELTMANN, Klaus-Dieter**
  **18609 Binz (DE)**

• **VON WOEDTKE, Thomas**
  **18519 Sundhagen (DE)**
• **STIEBER, Manfred**
  **17489 Greifswald (DE)**
• **KRÖMKER, Wilfried**
  **31675 Bückeburg (DE)**

(74) Vertreter: **Gerstein, Hans Joachim**
**Gramm, Lins & Partner**
**Patent- und Rechtsanwälte PartGmbB**
**Freundallee 13 a**
**30173 Hannover (DE)**

(56) Entgegenhaltungen:
WO-A1-00/67805      WO-A1-2010/066667
WO-A1-2014/135254      US-A1- 2013 272 929

**Beschreibung**

Die Erfindung betrifft eine Desinfektionsvorrichtung zur Plasmadesinfektion von Oberflächen mit einem Plasmagenerator zur Erzeugung eines desinfizierenden Plasmagasstroms und mit einem mit dem Plasmagenerator in kommunizierender Verbindung stehenden, mindestes teilweise geschlossenen Desinfektionsbereich, der zur Aufnahme der zu desinfizierenden Oberfläche ausgebildet ist.

Die Desinfektionsvorrichtung hat einen Aerosolgenerator zur Erzeugung eines wässrigen Partikel enthaltenen Aerosolstroms, wobei der Aerosolgenerator mit dem Plasmagenerator in kommunizierender Verbindung steht, um den mit dem Aerosolstrom vermischten Plasmastrom im Desinfektionsbereich auf die zu desinfizierende Oberfläche zu leiten.

Zur Desinfektion von Oberflächen, insbesondere zur Handdesinfektion in medizinischen Einrichtungen werden Desinfektionslösungen verwendet, die z. B. auf die zu desinfizierenden Hände aufgetragen werden.

EP 2 223 704 A1 beschreibt eine Einrichtung zur Desinfektion z. B. von Händen mit einem nicht-thermischen Plasma, das in ein einseitig offenes Gehäuse einströmt.

Weiterhin ist aus WO 2011/023478 A1 bekannt, eine flächige Behandlung von Bereichen menschlicher oder tierischer Haut- bzw. Schleimhautoberflächen mit kalten Atmosphärendruckplasmen vorzunehmen, um auf diese Weise Erkrankungen zu behandeln. Ein Elektrodensystem zur Erzeugung einer dielektrisch behinderten Oberflächenentladung besteht einerseits aus flexiblen Materialien, das sich an gekrümmte Oberflächen anschmiegen lässt, und hat andererseits eine äußere, elektrisch leitende Fläche, die als geerdete Elektrode verwendet und so strukturiert ist, dass sich in den freibleibenden Zwischenräumen der Struktur dielektrisch behinderte Oberflächenentladungen ausbilden können. Durch die Ausführung als anschmiegsame Manschette erfolgt eine Abdeckung des behandelten Areals und damit ein Schutz vor Austrocknung.

WO 2011/018423 A2 offenbart ein Verfahren und eine Vorrichtung zur Behandlung lebender Zellen mittels eines Plasmas, wobei neben den Einrichtungen zur Plasmaerzeugung weiterhin Einrichtungen zur Mischung und zum Transport von Wirkstoffen zur Beeinflussung des Stoffwechsels der Zellen vorgesehen sind. Damit ist es möglich, auf lebende Zellen mittels eines Plasmas Substanzen einwirken zu lassen. Durch die Wechselwirkung zwischen Plasma und Gewebe kommt es zu einer Änderung der Zusammensetzung der Lipidstrukturen, so dass kurzzeitig ein Eintritt der topisch applizierten Substanzen in dem Bereich der lebenden Zellen ermöglicht wird.

US 6,706,243 B1 offenbart eine Vorrichtung zur Handreinigung, bei der optional ein Plasmagasstrom in einen Handreinigungsraum geleitet werden kann. Zusätzlich werden die Hände mit einem durch eine Ionenquelle geleiteten Luftstrom unter Druck gereinigt. Eine Reinigungslösung wird in einen Gasstrom gepumpt. Der mit Reinigungslösung vermischte Gasstrom wird vernebelt und in den Plasmagenerator geleitet.

US 2013/0272929 A1 beschreibt eine Desinfektionsvorrichtung mit einer Fluidquelle und einem Plasmagenerator zur Erzeugung nicht-thermischen Plasmas. Das Fluid wird mit Plasma aktiviert, indem das plasmaaktivierte Fluid aus einer Düse in den Plasmaerzeugungsraum zwischen Elektroden geleitet wird.

WO 2014/135254 A1 offenbart ein Verfahren und eine Vorrichtung zur Reinigung eines Gegenstands für Desinfektionszwecke, bei dem ein Trägergas und ein Nebel einer Behandlungsflüssigkeit erzeugt und durch einen Plasmagenerator geleitet wird. Dabei wird eine Wechselwirkung der vernebelten Behandlungsflüssigkeit mit dem Plasma zur Herstellung aktivierten Nebels genutzt.

WO 00/67805 A1 offenbart eine Vorrichtung zur Handreinigung, bei der eine Reinigungslösung mit einem Gasstrom vermischt und durch einen Plasmagenerator geleitet wird. Die Handdesinfektion erfolgt zweistufig zunächst mit einem nicht mit Aerosolen versetzten trockenen Plasmagasstrom und anschließend mit Hilfe der im Hochspannungsfeld aktivierten Reinigungslösung.

Ausgehend hiervon ist es Aufgabe der vorliegenden Erfindung eine verbesserte Desinfektionsvorrichtung zur Plasmadesinfektion von Oberflächen und ein verbessertes Verfahren zur Plasmadesinfektion von Oberflächen zu schaffen, das eine effizientere und hautschonendere Desinfektion insbesondere von Zellen und Gewebe ermöglicht.

Die Aufgabe wird durch die Desinfektionsvorrichtung mit den Merkmalen des Anspruchs 1 und durch das Plasmadesinfektionsverfahren mit den Merkmalen des Anspruchs 12 gelöst. Vorteilhafte Ausführungsformen sind in den Unteransprüchen beschrieben.

Es wird vorgesehen, dass der Aerosolgenerator an den Plasmagasstrom-Ausgang des Plasmagenerators gekoppelt ist. Der vom Plasmagenerator erzeugte Plasmagasstrom wird dann mit dem Aerosolstrom des Aerosolgenerators vermischt und in den Desinfektionsbereich geleitet. Dadurch werden die Aerosole nicht durch den Plasmagenerator selbst beeinträchtigt. Insbesondere wird hierdurch vermieden, dass es im Plasmagenerator durch das Wasser-LuftGemisch zu einem Quenching des erzeugten Plasmas kommt, d.h. dass die Intensität des Plasmas durch wässrige Partikel wesentlich reduziert wird bzw. das Plasma partiell gelöscht wird und damit an Wirkung verliert. Besonders vorteilhaft ist es dabei, wenn der Plasmagasstrom durch das Volumen des Aerosolgenerators geleitet wird, da hierdurch eine hinreichende Verweildauer sichergestellt wird, die eine Modifizierung der Aerosole mittels des Plasmagasstroms gewährleistet. Insofern sollte das Volumen und die Durchflusszeit aufeinander abgestimmt werden.

Es hat sich herausgestellt, dass durch ein solches Durchleiten des Plasmagasstroms durch den Aerosolgenerator ein höherer Wirkungsgrad hinsichtlich der Desinfektion erzielt wird, als bei einem Leiten von Aerosol durch das Plasma.

Durch die Vermischung von wässrigen Aerosolpartikeln mit einem Plasmastrom und gleichzeitiger Oberflächendesinfektion mit dem wässrige Aerosolpartikel enthaltenen Plasmastrom gelingt es, die Desinfektion insbesondere von Hautgewebe überaus schonend und effektiv sowie prozesssicher durchzuführen. Es hat sich gezeigt, dass über die reine Plasmadesinfektion von Hautgewebe hinaus mittels der wässrigen Aerosolpartikel z. B. Pflegestoffe mit aufgebracht werden können, die den schädlichen Einfluss des Plasmas auf die Haut reduzieren und pflegend wirken und gleichzeitig zu einem effizienten und prozesssicheren Desinfektionsprozess beitragen.

Der Aerosolstrom und der Plasmagasstrom sind vorzugsweise so gewählt, dass der pH-Wert der auf die zu desinfizierende Oberfläche auftreffenden wässrigen Aerosolpartikel beeinflusst wird. Die Beeinflussung sollte vorzugsweise so erfolgen, dass der pH-Wert, der ansonsten bei destilliertem Wasser im Bereich von pH 5 bis 6 liegt, in Richtung des sauren Bereichs verschoben wird. Denkbar ist z.B. ein Aerosol, das selbst auch eine desinfizierende Wirkung hat. Besonders vorteilhaft ist es jedoch, wenn als Fluid für den Aerosolstrom reines Wasser ohne jegliche Zusatzstoffe, wie Reinigungslösung, Duftstoffe oder Pflegemittel verwendet wird. Dies resultiert in einem günstigen pH Bereich, der nicht zu sauer ist, d.h. im Bereich von etwa 3,5 bis 4 liegt.

Unabhängig von dem mit Wasser-Aerosolen (umfassend Wasserdampf) vermischten Plasmagasstrom können separat, vorzugsweise zeitlich nach der Plasmabehandlung Pflegemittel oder Duftstoffe appliziert werden. Damit kann dem Ozongeruch der behandelten Oberfläche entgegengewirkt und die Oberfläche zusätzlich gepflegt werden, z.B. mit Handcreme. Die Zuführung erfolgt vorzugsweise in dem Behandlungsraum oberhalb des Zuführbereichs für den Plasmagasstrom, d.h. angrenzend an die Öffnung zum Einführen der zu behandelnden Oberfläche. Die Düsenöffnungen für das Eindüsen von Duft- und/oder Pflegestoffen werden auf diese Weise bei der Plasmabehandlung gleich mit desinfiziert (entkeimt).

Besonders vorteilhaft ist es, wenn der Aerosolgenerator eine Vernebelungseinheit zum Vernebeln einer Flüssigkeit hat. Eine solche Flüssigkeit kann insbesondere eine emulsionshaltige Flüssigkeit sein, wie z. B. Emulsionen mit hautpflegenden Substanzen. Durch die Vernebelung einer solchen emulsionshaltigen Flüssigkeit gelingt es, die hautpflegenden Substanzen in eine in Verbindung mit der Plasmadesinfektion geeigneten Form mit dem Plasmastrom zu vermischen und auf die zu desinfizierende Oberfläche aufzutragen.

Besonders vorteilhaft ist es, wenn die Vernebelungseinheit einen Ultraschallvernebler hat. Es hat sich überraschend gezeigt, dass mit einem solchen Ultraschallvernebler hinreichend kleine Tröpfchengrößen erzeugt werden können, die in Verbindung mit dem Plasmastrom einen verbesserten Desinfektions- und Pflegeeffekt herbeiführen. Die durchschnittliche Tröpfchengröße bei der Ultraschallvernebelung liegt im Durchschnitt unterhalb von 5 $\mu$m und beträgt besonders bevorzugt durchschnittlich etwa 2 bis 4 $\mu$m. Diese Durchschnittswerte werden aus der Tröpfchengröße ermittelt, die sich bei mindestens 60 % und bevorzugt mindestens 80 % der gemessenen Tröpfchen maximal einstellt. Neben der sehr geringen Tröpfchengröße hat ein Ultraschallvernebler den Effekt, Aerosole mit hoher Viskosität zu erzeugen. Auch dies trägt zu der verbesserten Desinfektionswirkung in Verbindung mit einem Plasmastrom bei. Durch die Ultraschallvernebelung gelingt somit eine verbesserte Wechselwirkung der Aerosole mit dem Plasmastrom und im Ergebnis ein verbessertes Ergebnis der Oberflächendesinfektion. Eine solche Aerosolqualität lässt sich u.U. aber auch mit einer anderen Art der Vernebelung, wie z.B. einer mechanischen Vernebelung erzielen.

Vorteilhaft ist z.B. eine molekulare Anfeuchtung des Plasmagasstroms. Dies lässt sich mit einer angefeuchteten Befeuchtungsfläche erreichen, an der ein Luftstrom zur Anfeuchtung vorbeistreicht. Dieser Luftstrom ist vorzugsweise der Plasmagasstrom selbst.

Der Wirkungsgrad des Plasmagasstroms kann dadurch erhöht werden, dass der dem Plasmagenerator zugeführte Gasstrom (Luftstrom) vorgetrocknet wird. Hierzu ist dem Einlass des Plasmagenerators ein Lufttrockner (z.B. eine mit Silikagel gefüllte Kartusche) vorgeschaltet.

Als vorteilhaft hat es sich herausgestellt, wenn der Gasmengenstrom für das Plasmagas auf etwa 2 bis 20 Standardliter pro Minute, d.h. 2-20 slpm bzw. 3,3775 bis 33,775 $\dfrac{Pa \cdot m^3}{s}$ eingestellt wird. Die Aerosolmenge sollte dann etwa 0,5 bis 5 ml / min betragen. Das Verhältnis von Plasmagas zu Aerosolmenge liegt vorzugsweise im Bereich von etwa 400 zu 1 bis 40.000 zu 1.

Die Frequenz des Aerosolgenerators sollte bevorzugt mehr als 100 kHz betragen. Es hat sich herausgestellt, dass sich das Desinfektionsergebnis mit höherer Frequenz des Ultraschallverneblers von über 850 kHz verbessern lässt. Bevorzugt liegt die Frequenz im Bereich von 1.200 kHz bis 3.000 MHz.

Die Spannungsversorgung des Plasmagenerators sollte bei einer Frequenz der Hochspannung im Bereich von etwa 400 Hz bis 27,12 MHz oder mit Gleichspannung (DC) erfolgen.

Die Desinfektionsvorrichtung hat vorzugsweise ein Gebläse (oder eine Pumpe, wie z.B. eine Membranpumpe), das in kommunizierender Verbindung mit dem Desinfektionsbereich steht, um einen Luftstrom in den Desinfektionsbereich zu führen. Bei diesem Luftstrom kann es sich um den aerosolhaltigen Plasmastrom handeln, der mittels des Gebläses in den Desinfektionsbereich zwangsweise eingeströmt wird. Denkbar ist aber auch, dass ein zusätzlicher Luftstrom neben dem aerosolhaltigen Plasmastrom in den Desinfektionsbereich geführt wird.

Besonders vorteilhaft ist es, wenn das Gebläse (oder Pumpe) mit einer Heizeinrichtung zur Erwärmung des vom Gebläse bewirkten Luftstroms verbunden ist. Auf diese Weise wird die Wirktemperatur auf die zu desinfizierende Oberfläche erhöht, was die desinfizierende Wirkung des aerosolhaltigen Plasmastroms verbessert.

Das Gebläse (oder Pumpe) besteht in besonders vorteilhafter Weise in kommunizierender Verbindung mit dem Plasmagenerator, um einen vom Gebläse bewirkten Luftstrom durch den Plasmagenerator durchzuleiten. Dieser Luftstrom wird durch den Plasmagenerator in einen Plasmastrom überführt, der mit Aerosolen vermischt ist.

Der Desinfektionsbereich ist vorzugsweise zur Aufnahme mindestens einer zu desinfizierenden Hand einer Person und zur Desinfektion der Oberfläche der mindestens zu der in dem Desinfektionsbereich aufgenommenen Hand ausgebildet. Damit ist das Desinfektionsverfahren in besonders vorteilhafter Weise zur Handdesinfektion geeignet, die z. B. in Krankenhäusern regelmäßig von dem Klinikpersonal durchgeführt werden muss. Ein weiteres Einsatzgebiet sind andere vergleichbare Einrichtungen, in denen höhere Anforderungen an die Hygiene bestehen, wie Toilettenanlagen, Schwimmbäder, Arztpraxen, etc. Eine solche Desinfektionsvorrichtung erlaubt eine schnelle, zuverlässige und hautschonende Handdesinfektion, bei der berührungslos mit Hilfe der Aerosole auch Pflegesubstanzen auf die Haut aufgetragen werden. Die berührungslose Handdesinfektion im Desinfektionsbereich stellt eine Desinfektion auch schwer zugänglicher Bereiche unabhängig vom individuellen Reinigungsprozedere der einzelnen Personen sicher. Damit lässt sich prozesssicher eine zuverlässige Handdesinfektion erreichen.

Der Desinfektionsbereich sollte nach Einbringen der zu desinfizierenden Oberfläche möglichst gut abgeschlossen sein, um eine zuverlässige Oberflächenbehandlung ungestört unter Standardbedingungen sicherzustellen. Hierzu ist es vorteilhaft, wenn der ansonsten abgeschlossene Desinfektionsbereich eine Öffnung zum Einbringen der zu behandelnden Oberfläche, z.B. einer Hand, hat, die mit Hilfe mindestens einen Luftvorhangs abgeschlossen wird. Der Luftvorhang wird durch einen Luftstrom gebildet, der durch Luftdüsen geleitet wird. Vorteilhaft sind hierzu Luftmesser geeignet, bei denen die austretende Luft eine schmale und hinreichend breite Form annimmt und einen Luftvorhang mit annähernd gleicher Länge am Anfang und am Ende bewirkt. Der Luftspalt liegt vorzugsweise im Bereich zwischen 0,5 bis 0,8 mm.

Die Erfindung wird nachfolgend anhand von Ausführungsbeispielen mit den beigefügten Zeichnungen näher erläutert. Es zeigen:

Figur 1 - Skizze einer ersten Ausführungsform einer Desinfektionsvorrichtung zur Plasmadesinfektion von Oberflächen;

Figur 2 - Skizze einer Desinfektionsvorrichtung zur Plasmadesinfektion;

Figur 3 - Skizze einer anderen Desinfektionsvorrichtung zur Plasmadesinfektion;

Figur 4 - Skizze einer zweiten Ausführungsform einer Desinfektionsvorrichtung zur Plasmadesinfektion;

Figur 5 - Skizze einer dritten Ausführungsform einer Desinfektionsvorrichtung zur Plasmadesinfektion;

Figur 6 - Skizze eines Aerosolgenerators mit Sinterplatten zur molekularen Anfeuchtung des Plasmagasstroms.

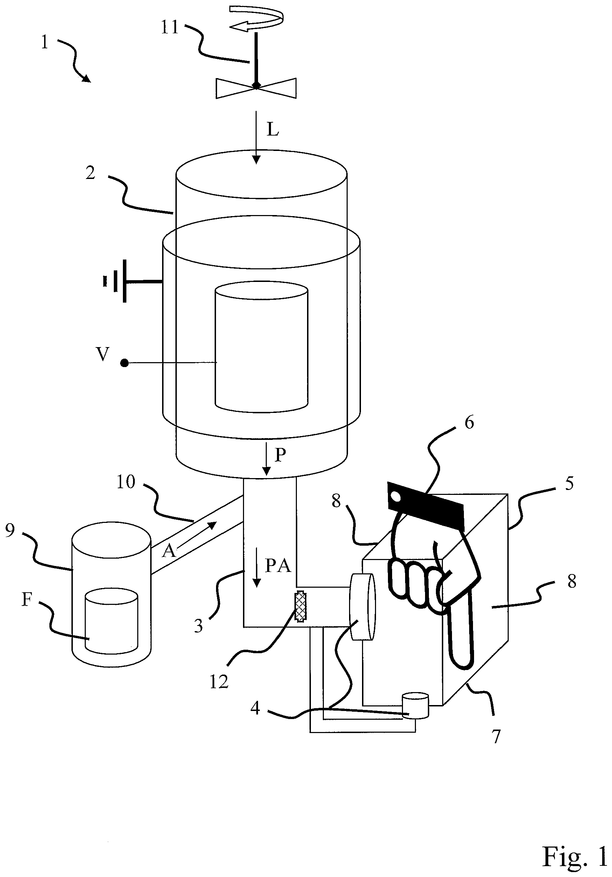

Figur 1 lässt eine Skizze einer ersten Ausführungsform einer Desinfektionsvorrichtung 1 zur Plasmadesinfektion von Oberflächen, insbesondere von menschlichen Händen 6 einer Person erkennen. Die Desinfektionsvorrichtung 1 hat einen Plasmagenerator 2, mit dem ein desinfizierender Plasmagasstrom P erzeugt wird. Der Plasmagasstrom P wird über eine Plasmazuleitung 3 und Düsen 4 in einen Desinfektionsbereich 5 eingeleitet, in den die zu desinfizierende Oberfläche, wie beispielsweise die Hand 6 einer Person, gehalten werden kann. Der Desinfektionsbereich 5 ist vorzugsweise als nur auf einer Seite offener Behälter ausgestaltet, dessen Seitenwände 8 die Düsen 4 aufweisen. Denkbar ist auch, dass weitere Düsen 4 in dem Boden 7 des Desinfektionsbereichs 5 vorhanden sind. Der Desinfektionsbereich 5 ist vorzugsweise rechteckförmig mit relativ nah nebeneinander liegenden Seitenwänden 8, zwischen denen die Hand 6 flach eingebracht werden kann.

Deutlich wird weiterhin, dass an den Ausgang des Plasmagenerators 2 in die Plasmazuleitung 3 ein Aerosolgenerator 9, vorzugsweise in Form eines Ultraschallverneblers, geschaltet ist. Der Ausgang des Aerosolgenerators 9 steht über eine Aerosolleitung 10 in kommunizierender Verbindung mit der Plasmaleitung 3 am Ausgang des Plasmagenerators 2. Auf diese Weise kann ein Aerosolstrom A, der durch Vernebelung eines Fluids F durch den Ultraschallvernebler erzeugt wird, mit dem Plasmastrom P vermischt werden. Dieser aerosolhaltige Plasmastrom P wird dann über die Düsen 4 in den Desinfektionsbereich 5 eingebracht.

Die hierzu erforderliche Strömung wird mit Hilfe eines Gebläses 11 erzeugt, dass in kommunizierender Verbindung mit dem Eingang des Plasmagenerators 2 steht, um einen Luftstrom L in den Innenraum des Plasmagenerators 2 zu führen. Dieser Luftstrom L wird dann durch den Plasmagenerator 2 in einen Plasmagasstrom PA überführt.

Als Plasmagenerator 2 kann z. B. eine Plasmaionenquelle zur Erzeugung einer dielektrisch behinderten Entladung dienen, wie sie an sich hinreichend aus dem Stand der Technik bekannt ist. Geeignet sind insbesondere Plasmageneratoren 2, die ein nicht-thermisches Plasma erzeugen. Denkbar ist, dass die Plasmageneratoren 2 Oberflächen-DBE-Quellen oder Volumen-DBE-Quellen (DBE = dielektrisch behinderte Entladung) nutzen. Alternativ ist auch ein Plasmagenerator 2 basierend auf einem Plasmajet-Verfahren denkbar. Möglich sind auch jet-artige Plasmen, Bogenplasmen, Plasmatorches, kapazitiv oder induktiv angeregte Plasmen im oder nahe dem Normaldruckbereich sowie durch Coronaentladung erzeugte Plasmen.

Nicht-thermisches Plasma enthält in der Regel Ozon und ist damit oxidierend. Nicht-thermisches Plasma eignet sich damit besonders zur Oberflächendesinfektion.

Der Plasmagenerator kann z.B. zwei ineinanderliegende (z.B. röhrenförmige) Elektroden haben, von denen eine mit Hochspannung V beaufschlagt wird und die andere mit Massepotential verbunden ist.

Der Aerosolgenerator 9 ist vorzugsweise als Ultraschallvernebler ausgebildet. Die von einem solchen Aerosolgenerator 9 erzeugten Aerosole A haben vorzugsweise eine durchschnittliche Partikelgröße im Bereich von maximal 15 $\mu$m und bevorzugt maximal 12 $\mu$m. Die durchschnittliche Partikelgröße liegt vorzugsweise im Bereich von 1 bis 15 $\mu$m und besonders bevorzugt im Bereich von 4 bis 12 $\mu$m. Größere Oberflächen der Aerosolpartikel haben den Nachteil, dass diese mehr Ozon aufnehmen und damit die Wirkung des Plasmastroms P bzw. PA signifikant reduzieren.

Optional kann zusätzlich eine Heizeinrichtung 12 in den Luft- und/oder Plasmastrom L bzw. P oder sogar in den aerosolhaltigen Plasmastrom PA eingebracht sein. Damit lässt sich eine geeignete Temperatur durch Abkühlen des Plasmagasstroms P oder ggf. durch Aufheizen erzielen.

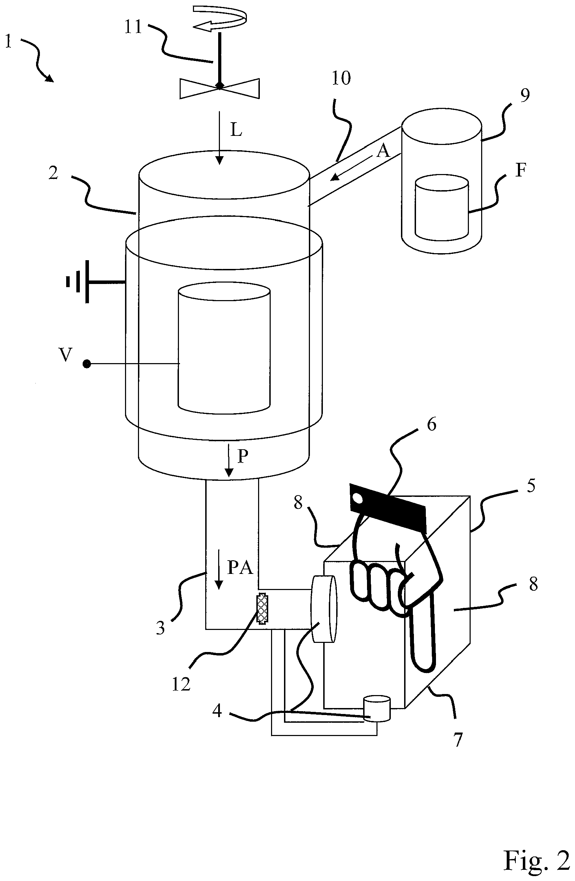

Figur 2 lässt eine Desinfektionsvorrichtung 1 erkennen. Diese ist mit der ersten Ausführungsform der Desinfektionsvorrichtung 1 vergleichbar, so dass im Wesentlichen auf das Vorhergesagte verwiesen werden kann. Im Unterschied zur erfindungsgemäßen ersten Ausführungsform ist der Aerosolgenerator 9 mit dem Eingang des Plasmagenerators 2 gekoppelt. Damit wird der Aerosolstrom A im Eingangsbereich des Plasmagenerators 2 mit dem Luftstrom L vermischt und durch den Plasmagenerator 2 in einen aerosolhaltigen Plasmagasstrom PA überführt.

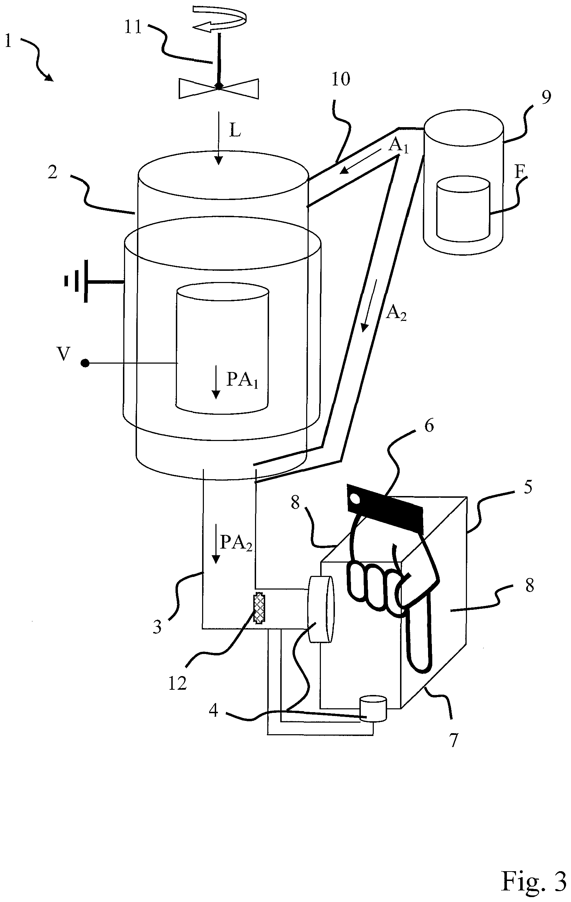

Figur 3 lässt eine Desinfektionsvorrichtung 1 erkennen, die eine Kombination der ersten und zweiten Ausführungsform gemäß Figuren 1 und 2 ist. Dabei wird ein Teil-Aerosolstrom $A_1$ in den Eingang des Plasmagenerators 2 und ein anderer Teil-Aerosolstrom $A_2$ in den Ausgang des Plasmagenerators 2 geführt. Der vom Plasmagenerator 2 erzeugte bereits aerosolhaltige Plasmagasstrom $PA_1$ wird durch noch weiteres Zuführen des Aerosolstroms $A_2$ weiter aufbereitet.

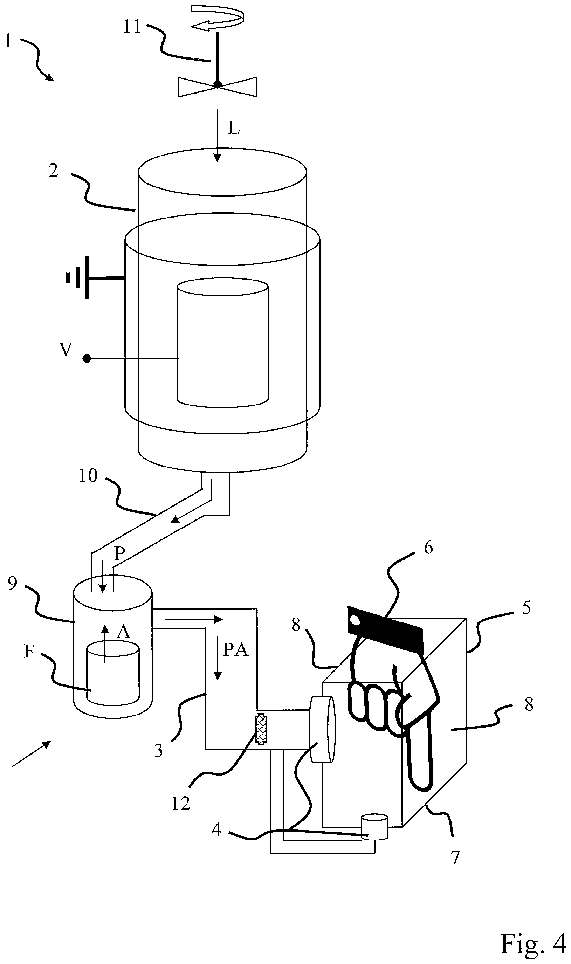

Figur 4 zeigt eine zweite Ausführungsform einer Desinfektionsvorrichtung 1, die im Prinzip mit den oben beschriebenen Ausführungsformen vergleichbar ist. Bei dieser Ausführungsform wird der Plasmagasstrom P allerdings durch den Aerosolgenerator 9 geführt und das Aerosol bei der Erzeugung des Aerosols z.B. durch Ultraschallvernebelung direkt in den Plasmagasstrom P eingebracht, um den aerosolhaltigen Plasmagasstrom PA zu erhalten.

Der in den Figuren 1 bis 4 skizzierte runde Elektrodenquerschnitt ist bevorzugt. Optimal sind andere Querschnitte, wie insbesondere rechteckig oder quadratisch denkbar.

Der Desinfektionsprozess kann durch Regelung der Prozessparameter der Flussgeschwindigkeit des Luftstroms L, des Plasmastroms P und/oder des Aerosolstroms A und deren Teilströme $A_1$, $A_2$, der Elektrodenabstände, der Tröpfchengröße der Aerosole, des Sättigungsgrades der Aerosole im Plasmastrom P, des elektrischen Stroms und der elektrischen Spannung zur Plasmaerzeugung etc. optimiert werden.

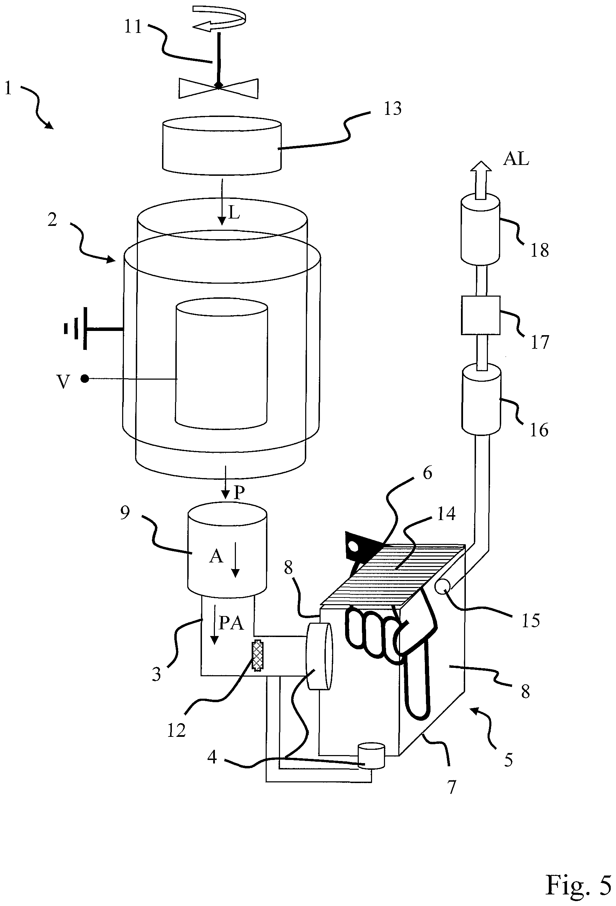

Figur 5 lässt eine Skizze einer dritten Ausführungsform einer Desinfektionsvorrichtung 1 zur Plasmadesinfektion erkennen. Diese basiert im Prinzip auf der ersten Ausführungsform gemäß Figur 1, sodass auf die dortigen Ausführungen verwiesen werden kann. Sie ist allerdings dahingehend ergänzt, dass der in den Plasmagenerator 2 geleitete Luftstrom L mit Hilfe eines Lufttrockners 13 zum Vortrocknen des Luftstroms L geleitet wird. Als Lufttrockner eignet sich z.B. eine mit Silikagel gefüllte Kartusche, deren Einlass mit einer Membranpumpe zum Einpumpen von Luft aus der Atmosphäre in den Lufttrockner 13 verbunden ist. Der Auslass der Kartusche ist dann mit dem Einlass des Plasmagenerators 2 z.B. über eine Schlauchleitung verbunden.

Der Auslass des Plasmagenerators 2 steht z.B. über eine Schlauchleitung in kommunizierender Verbindung mit dem Aerosolgenerator 9 derart, dass der Plasmagasstrom P durch den Aerosolgenerator 9 geleitet wird. Hierbei wird der Plasmagasstrom P mit Aerosol A angefeuchtet, um einen feuchten Plasmagasstrom PA zu bilden. Dieser wird dann in den Behandlungsraum, d.h. den Desinfektionsbereich 5 geleitet.

Der durch Seitenwände 8 umfangsseitig und durch einen Boden 7 abgeschlossene Desinfektionsbereich 5 ist nur an einer Seite zum Einführen des Hand 6 einer Person geöffnet. Diese offene Seite hat mit Hilfe geeigneter Luftdüsen einen Luftvorhang 14, durch den trotz eingeführter Hand 6 und herausragendem Arm einer Person der Desinfektionsbereich 5, d.h. der Behandlungsraum hinreichend abgeschlossen ist. Die Luftdüsen sind vorzugsweise als Luftmesser ausgeführt, das einen austretenden Luftstrom mit einer sehr schmalen und beinahe beliebig breiten Form entsprechend der Breite der Öffnung des Behandlungsraums erzeugt. Der Luftspalt des Luftvorhangs ist vorzugsweise so schmal, dass er im Bereich von 0,5 bis 0,8 mm liegt. Die für den Luftvorhang 14 erforderliche Luftmenge wird mit einem definierten Druck durch das mindestens eine Luftmesser geleitet. Das Luftmesser hat dabei einen langen, schmalen Schlitz und erzeugt einen messerartigen Luftvorhang 14 mit einer konstant hohen Luftgeschwindigkeit. Der Anfang und das Ende des Luftvorhangs 14 sind relativ gleich lang.

Weiterhin ist bei dieser Ausführungsform vorgesehen, dass der Desinfektionsbereich 5, d.h. der Behandlungsraum einen Auslass 15 hat, mit dem ein Kondensat-Abscheider 16 und ein weiterer Lufttrockner 17 kommunizierend z.B. über eine Schlauchleitung verbunden ist. An dem Ausgang des Lufttrockners 17 ist eine Luftabsaugpumpe 19 z.B. in Form einer Membranpumpe gekoppelt, um in den Behandlungsraum 5 eingeführte plasmaaktivierte Luft nach der Behandlung wieder aus dem Behandlungsraum 5 abzuziehen. Mit dem Kondensat-Abscheider 16, der z.B. mit einer Peltier-Kühlung realisiert sein kann, wird zur Schonung des Lufttrockners 17 der Feuchtegehalt reduziert. Die mit dem Lufttrockner 17 getrocknete Abluft wird dann einem Ozonkatalysator 18 zugeführt, um den Anteil von Ozon aus der Abluft AL zu reduzieren.

Anstelle eines Ozonkatalysators 18 ist auch eine andere Einrichtung geeignet, mit der der Ozonanteil reduziert werden kann, d.h. mit der Ozon aus dem Abgas weitestgehend vernichtet wird.

Als Aerosolgenerator 9 ist wie vorher beschrieben ein Ultraschall-Aerosolgenerator geeignet, insbesondere wenn dieser eine besonders feine Vernebelung mit einer durchschnittlichen Partikelgröße im Bereich von 4 μm und weniger sicherstellt. Denkbar ist aber auch eine Nutzung von Verdunstern, wie sie vom Wirkprinzip auch zur Raumluftbefeuchtung verwendet werden.

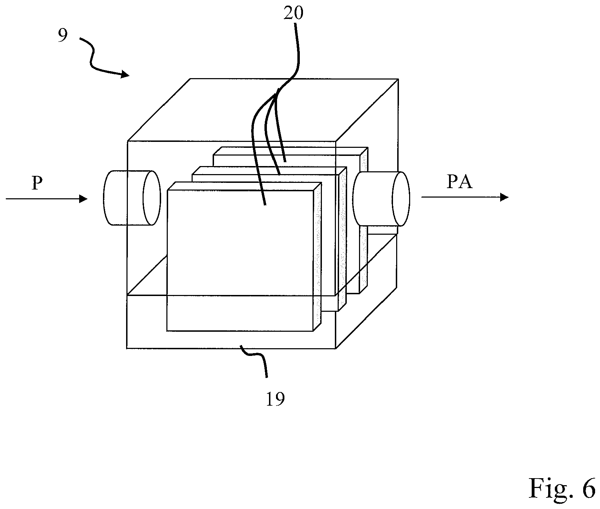

Besonders geeignet ist ein Aerosolgenerator 9 zur molekularen Anfeuchtung des Plasmagasstroms P dadurch, dass der Plasmagasstrom P an Befeuchtungsflächen 20 vorbeigeführt wird. Ein solcher Aerosolgenerator 9 mit mehreren Sinterplatten ist in Figur 6 skizziert. Die Befeuchtungsflächen 20 sind bspw. PE-Sinterplatten oder andere Filterplatten mit einer definierten Porosität, die in einer mit ionenfreiem oder ionenreduziertem Wasser gefüllten Wanne 19 stehen. Der aus der Wanne 19 herausragende Plattenabschnitt der mindestens einen Sinterplatte 20 wird dann von dem Plasmagasstrom P umströmt. Beim Vorbeistreichen des Plasmagasstroms P gibt die mindestens eine befeuchtete Sinterplatte 20 Feuchtigkeit an diesen Luftstrom ab. Der Plasmagasstrom P weist hierdurch eine relativ hohe relative Luftfeuchte auf. Das zur Befeuchtung der mindestens einen Sinterplatte 20 benutzte Fluid kann ohne Pumpen aufgrund der Kapillarwirkung der Sinterplatten 20 in die Wanne 19 durch die Pumpwirkung der Sinterplatten 20 eingeleitet werden. Die Befeuchtungsfläche 20 z.B. in Form einer PE-Platte aus Sintermaterial, welche Kapillare aufweist, steht vorzugsweise mit etwa 1/10 ihrer Höhe in einer Wanne 19 mit ionenfreiem Wasser. Die Sinterplatte 20 hat vorzugsweise eine Dicke von etwa 2 mm +/- 1 mm.

An dem Boden des Aerosolgenerators 9 ist vorzugsweise eine Heizung 12 zur Anwärmung des Fluids (vorzugsweise reines Wasser) vorhanden.

Der Desinfektionsbereich 5 ist in allen vorstehend beschriebenen Ausführungsformen vorzugsweise oberhalb des Aerosolgenerators 9 und des Plasmagenerators 2 angeordnet, so dass der mit Aerosol vermischte Plasmastrom PA entgegen der Schwerkraft aufwärts in den Desinfektionsraum 5 strömt. Dies hat zur Folge, dass Kondensat wieder in den Aerosolgenerator 9 zurückfließt und im Wesentlichen erst gar nicht in den Desinfektionsraum 5 gelangt.

**Patentansprüche**

1. Desinfektionsvorrichtung (1) zur Plasmadesinfektion von Oberflächen mit einem Plasmagenerator (2) zur Erzeugung eines desinfizierenden Plasmagasstroms (P) und mit einem mit dem Plasmagenerator (2) in Verbindung stehenden, mindestens teilweise geschlossenen Desinfektionsbereich (5), der zur Aufnahme der zu desinfizierenden Oberfläche ausgebildet ist, wobei die Desinfektionsvorrichtung (1) einen Aerosolgenerator (9) zur Erzeugung eines wässrigen Partikel enthaltenen Aerosolstroms (A) hat, wobei der Aerosolgenerator (9) mit dem Plasmagenerator (2) in Verbindung steht, um den mit dem Aerosolstrom (A) vermischten Plasmagasstrom (PA) im Desinfektionsbereich (5) auf die zu desinfizierende Oberfläche zu leiten, **dadurch gekennzeichnet, dass** der Aerosolgenerator (9) an den Plasmagasstrom-Ausgang des Plasmagenerators (2) gekoppelt ist und der vom Plasmagenerator (2) erzeugte Plasmagasstrom (P) mit dem Aerosolstrom (A) des Aerosolgenerators (9) vermischt und in den Desinfektionsbereich (5) geleitet wird.

2. Desinfektionsvorrichtung (1) nach Anspruch 1, **dadurch gekennzeichnet, dass** der Aerosolgenerator (9) eine Vernebelungseinheit zum Vernebeln eines Fluids (F), insbesondere einer emulsionshaltigen Flüssigkeit hat.

3. Desinfektionsvorrichtung (1) nach Anspruch 2, **dadurch gekennzeichnet, dass** die Vernebelungseinheit einen Ultraschallvernebler hat.

4. Desinfektionsvorrichtung (1) nach Anspruch 1, **dadurch gekennzeichnet, dass** der Aerosolgenerator (9) mindestens eine Befeuchtungsfläche (20) hat, die mit Fluid (F) zum Anfeuchten der Befeuchtungsfläche (20) in Verbindung steht und mit einem Luftstrom (L) beaufschlagbar so angeordnet ist, dass der an der mindestens einen Befeuchtungsfläche (20) vorbeistreichende Luftstrom (L) angefeuchtet wird und als Aerosolstrom (A) mit dem Plasmagasstrom (PA) vermischt wird.

**5.** Desinfektionsvorrichtung (1) nach Anspruch 4, **dadurch gekennzeichnet, dass** die mindestens eine Befeuchtungsfläche (20) als Kapillaren aufweisende Platte ausgebildet ist, die in einem Fluidbad steht und deren aus dem Fluidbad herausragender Plattenabschnitt mit einem Luftstrom (L) beaufschlagbar ist.

**6.** Desinfektionsvorrichtung (1) nach einem der vorhergehenden Ansprüche, **dadurch gekennzeichnet, dass** die Desinfektionsvorrichtung (1) ein Gebläse (11) hat, das in kommunizierender Verbindung mit dem Desinfektionsbereich (5) steht, um einen Luftstrom (L) in den Desinfektionsbereich (5) zu führen.

**7.** Desinfektionsvorrichtung (1) nach Anspruch 6, **dadurch gekennzeichnet, dass** das Gebläse (11) mit einer Heizeinrichtung (12) zur Erwärmung des vom Gebläse (11) bewirkten Luftstroms (L) verbunden ist.

**8.** Desinfektionsvorrichtung (1) nach Anspruch 6 oder 7, **dadurch gekennzeichnet, dass** das Gebläse (11) in Verbindung mit dem Plasmagenerator (2) zum Durchleiten eines vom Gebläse (11) bewirkten Luftstroms (L) durch den Plasmagenerator (2) steht.

**9.** Desinfektionsvorrichtung (1) nach einem der vorhergehenden Ansprüche, **dadurch gekennzeichnet, dass** der Desinfektionsbereich (5) zur Aufnahme mindestens einer zu desinfizierenden Hand (6) einer Person und zur Desinfektion der Oberfläche der mindestens in dem Desinfektionsbereich (5) aufgenommenen Hand (6) ausgebildet ist.

**10.** Desinfektionsvorrichtung (1)nach einem der vorhergehenden Ansprüche, **dadurch gekennzeichnet, dass** der Desinfektionsbereich (5) eine Öffnung zur Aufnahme der zu desinfizierenden Oberfläche hat, die mit einem Luftvorhang (14), der durch mindestens einen aus einer Luftdüse austretenden Luftstrom (L) gebildet wird, von der Umgebung abschließbar ist.

**11.** Desinfektionsvorrichtung (1) nach einem der vorhergehenden Ansprüche, **dadurch gekennzeichnet, dass** der Desinfektionsbereich (5) mit einer Luftabsauganordnung in kommunizierender Verbindung steht, wobei die Luftabsauganordnung zum Abführen des mit dem Aerosolstrom (A) vermischten Plasmagasstroms (P) der in den Desinfektionsbereich (5) eingeleitet ist, eingerichtet ist und einen Ozonkatalysator zur Neutralisation von im abgesaugten Luftstrom (L) befindlichem Ozon hat.

**12.** Verfahren zur Plasmadesinfektion von Oberflächen, insbesondere von Zellen und/oder von Gewebe, mit einem Plasmagenerator (2) zur Erzeugung eines desinfizierenden Plasmagasstroms (P), **gekennzeichnet durch** Vermischen eines wässrige Partikel enthaltenden Aerosolstroms (A) mit dem von dem Plasmagenerator (2) erzeugten Plasmagasstrom (P) und Leiten des wässrige Aersolpartikel enthaltenden Plasmagasstroms (PA) in eine mindestens teilweise geschlossene Desinfektionsbereich (5) auf die zu desinfizierende, in dem Desinfektionsbereich (5) aufgenommene Oberfläche.

**13.** Verfahren nach Anspruch 12, **gekennzeichnet durch** Ultraschallvernebelung einer Flüssigkeit und Einleiten des ultraschallvernebelten wässrigen Aerosolstroms (A) vor oder nach der Erzeugung eines Plasmagasstroms (P) in einen Plasmagasstrom (P) zum Vermischen des Aerosolstroms (A) mit dem Plasmagasstrom (P).

**14.** Verfahren nach Anspruch 12, **gekennzeichnet durch** molekulares Anfeuchten eines Luftstroms (L) zur Bildung des Aerosolstroms (A).

**15.** Verfahren nach einem der Ansprüche 12 bis 14, **gekennzeichnet durch** Vortrocknen des in den Plasmagenerator (2) eingeleiteten Luftstroms (L).

## Claims

**1.** A disinfection device (1) for plasma disinfection of surfaces, with a plasma generator (2) for generating a disinfecting plasma gas stream (P) and with an at least partly closed disinfection region (5) which is communicatively connected to the plasma generator (2) and which is embodied to receive the surface to be disinfected, wherein the disinfection device (1) has an aerosol generator (9) for generating an aerosol stream (A) containing aqueous particles, wherein the aerosol generator (9) is communicatively connected to the plasma generator (2) in order to guide the plasma gas stream (PA) mixed with the aerosol stream (A) onto the surface to be disinfected in the disinfection region (5), **characterized in that** the aerosol generator (9) is coupled to the plasma gas stream outlet of the plasma generator (2) and the plasma gas stream (P) generated by the plasma generator (2) is mixed with the aerosol stream (A) of the aerosol generator (9) and guided into the disinfection region (5).

**2.** The disinfection device (1) as claimed in claim 1, **characterized in that** the aerosol generator (9) has an atomizer unit for atomizing a fluid (F), more particularly an emulsion-containing liquid.

**3.** The disinfection device (1) as claimed in claim 2, **characterized in that** the atomizer unit has an ultrasonic atomizer.

4. The disinfection device (1) as claimed in claim 1, **characterized in that** the aerosol generator (9) has at least one humidifying surface (20), which is connected with fluid (F) for moistening the humidifying surface (20) and which is arranged actuatable by an air stream in such a way that the air stream sweeping past the at least one humidifying surface (20) is moistened and mixed with the plasma gas stream (PA) as aerosol stream (A).

5. The disinfection device (1) as claimed in claim 4, **characterized in that** the at least one humidifying surface (20) is embodied as a plate having capillaries, which plate stands in a fluid bath and the plate portion thereof protruding out of the fluid bath can be actuated by an air stream.

6. The disinfection device (1) as claimed in one of the preceding claims, **characterized in that** the disinfection device (1) has a fan (11), which is communicatively connected to the disinfection region (5) in order to guide an air stream (L) into the disinfection region (5).

7. The disinfection device (1) as claimed in claim 6, **characterized in that** the fan (11) is connected to a heating apparatus for heating the air stream caused by the fan.

8. The disinfection device (1) as claimed in claim 6 or 7, **characterized in that** the fan (11) is communicatively connected to the plasma generator (2) for guiding an air stream (L) caused by the fan (11) through the plasma generator (2).

9. The disinfection device (1) as claimed in one of the preceding claims, **characterized in that** the disinfection region (5) is embodied to receive at least one hand (6) to be disinfected of a person and to disinfect the surface of the hand (6) at least received in the disinfection region (5).

10. The disinfection device (1) as claimed in one of the preceding claims, **characterized in that** the disinfection region (5) has an opening for receiving the surface to be disinfected, which opening can be closed-off from the surroundings by an air curtain (14), which is formed by at least one air stream (L) emerging from an air nozzle.

11. The disinfection device (1) as claimed in one of the preceding claims, **characterized in that** the disinfection region (5) is communicatively connected to an arrangement for suctioning away air, wherein the arrangement for suctioning away air is configured to lead away the plasma gas stream (P) mixed with the aerosol stream (A) which is introduced into the disinfection region (5) and it has an ozone catalyzer for neutralizing ozone situated in the suctioned-away air stream (L).

12. A method for plasma disinfection of surfaces, more particularly of cells and/or tissue, with a plasma generator (2) for generating a disinfecting plasma gas stream (P), **characterized by** mixing an aerosol stream (A) containing aqueous particles with the plasma gas stream (P) generated by the plasma generator (2) and guiding the plasma gas stream (PA) containing aqueous aerosol particles into an at least partly closed disinfection region (5) onto the surface to be disinfected, which is received in the disinfection region (5).

13. The method as claimed in claim 12, **characterized by** ultrasonic atomization of a liquid and introducing the aqueous aerosol stream (A) atomized by ultrasound into a plasma gas stream (P) prior to or after the generation of a plasma gas stream (P) for the purposes of mixing the aerosol stream (A) with the plasma gas stream (P).

14. The method as claimed in claim 12, **characterized by** molecular moistening of an air stream for forming the aerosol stream (A).

15. The method as claimed in one of claims 12 to 14, **characterized by** pre-drying the air stream (L) introduced into the plasma generator (2).

## Revendications

1. Ensemble de désinfection (1) pour la désinfection de surfaces à l'aide d'un plasma, présentant un générateur (2) de plasma qui forme un écoulement (P) de plasma de désinfection et une zone de désinfection (5) communiquant avec le générateur (2) de plasma, configurée pour reprendre la surface à désinfecter et au moins en partie fermée, l'ensemble de désinfection (1) possédant un générateur (9) d'aérosol qui forme un écoulement (A) d'aérosol contenant des particules aqueuses, le générateur (9) d'aérosol communiquant avec le générateur (2) de plasma pour conduire sur la surface à désinfecter placée dans la zone de désinfection (5) l'écoulement (PA) de gaz à l'état de plasma mélangé avec l'écoulement (A), **caractérisé en ce que** le générateur (9) d'aérosol est accouplé à la sortie d'écoulement de gaz à l'état de plasma du générateur (2) de plasma et **en ce que** l'écoulement (P) de gaz à l'état de plasma formé par le générateur (2) de plasma est mélangé avec l'écoulement (A) d'aérosol du générateur (9) d'aérosol et est amené dans la partie de désinfection (5).

**2.** Ensemble de désinfection (1) selon la revendication 1, **caractérisé en ce que** le générateur (9) d'aérosol possède une unité de formation de brouillard qui forme un brouillard de fluide (F), en particulier d'un fluide contenant une émulsion.

**3.** Ensemble de désinfection (1) selon la revendication 2, **caractérisé en ce que** l'unité de formation de brouillard possède un formateur de brouillard à ultrasons.

**4.** Ensemble de désinfection (1) selon la revendication 1, **caractérisé en ce que** le générateur (9) d'aérosol possède au moins une surface d'humidification (20) qui communique avec le fluide (F) pour humidifier la surface d'humidification (20) et peut être alimentée par un écoulement d'air (L) disposé de telle sorte que l'écoulement d'air (L) qui balaye la ou les surfaces d'humidification (20) est humidifié et est mélangé sous la forme d'un écoulement (A) d'aérosol avec l'écoulement (PA) de gaz à l'état de plasma.

**5.** Ensemble de désinfection (1) selon la revendication 4, **caractérisé en ce que** la ou les surfaces d'humidification (20) est configurée comme plaque présentant les capillaires, placée dans un bain de fluide, un écoulement d'air (L) pouvant être appliqué sur la partie de la plaque qui déborde du bain de fluide.

**6.** Ensemble de désinfection (1) selon l'une des revendications précédentes, **caractérisé en ce que** l'ensemble de désinfection (1) possède un ventilateur (11) qui communique avec la zone de désinfection (5) pour amener un écoulement d'air (L) dans la zone de désinfection (5).

**7.** Ensemble de désinfection (1) selon la revendication 6, **caractérisé en ce que** le ventilateur (11) est relié à un dispositif de chauffage (12) qui chauffe l'écoulement d'air (L) formé par le ventilateur (11).

**8.** Ensemble de désinfection (1) selon les revendications 6 ou 7, **caractérisé en ce que** le ventilateur (11) communique avec le générateur (2) de plasma pour faire passer à travers le générateur (2) de plasma un écoulement d'air (L) formé par le ventilateur (11).

**9.** Ensemble de désinfection (1) selon l'une des revendications précédentes, **caractérisé en ce que** la zone de désinfection (5) est configurée pour reprendre au moins la main (6) à désinfecter d'une personne et pour désinfecter la surface de la ou des mains (6) placées dans la zone de désinfection (5).

**10.** Ensemble de désinfection (1) selon l'une des revendications précédentes, **caractérisé en ce que** la zone de désinfection (5) possède une ouverture qui reprend la surface à désinfecter et qui peut être séparée de l'environnement par un rideau d'air (14) formé par au moins un écoulement d'air (L) qui sort d'une tuyère à air.

**11.** Ensemble de désinfection (1) selon l'une des revendications précédentes, **caractérisé en ce que** la zone de désinfection (5) communique avec un ensemble d'aspiration d'air, l'ensemble d'aspiration d'air étant conçu pour évacuer l'écoulement (P) de gaz à l'état de plasma mélangé avec l'écoulement (A) d'aérosol qui est amené dans la zone de désinfection (5) et possède un catalyseur à ozone qui neutralise l'ozone présent dans l'écoulement d'air (L) aspiré.

**12.** Procédé de désinfection de surfaces par un plasma, en particulier de cellules et/ou de tissus, présentant un générateur (2) de plasma qui forme un écoulement (P) de gaz à l'état de plasma servant à la désinfection, **caractérisé par**
le mélange d'un écoulement (A) d'aérosol contenant des particules aqueuses avec l'écoulement (P) de gaz à l'état de plasma formé par le générateur (2) de plasma et
l'amenée de l'écoulement (PA) de gaz à l'état de plasma contenant les particules aqueuses d'aérosol dans une zone de désinfection (5) au moins en partie fermée, sur la surface à désinfecter placée dans la zone de désinfection (5).

**13.** Procédé selon la revendication 12, **caractérisé par** la formation à l'aide d'ultrasons d'un brouillard de liquide et l'introduction de l'écoulement (A) d'aérosol aqueux transformé en brouillard par ultrasons avant ou après la formation d'un écoulement (P) de gaz à l'état de plasma dans un écoulement (P) de gaz à l'état de plasma, pour mélanger l'écoulement (A) d'aérosol avec l'écoulement (P) de gaz à l'état de plasma.

**14.** Procédé selon la revendication 12, **caractérisé par** l'humidification moléculaire d'un écoulement d'air (L) en vue de former l'écoulement (A) d'aérosol.

**15.** Procédé selon l'une des revendications 12 à 14, **caractérisé par** le pré-séchage de l'écoulement d'air (L) introduit dans le générateur (2) de plasma.

Fig. 1

Fig. 2

Fig. 3

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2223704 A1
- WO 2011023478 A1
- WO 2011018423 A2
- US 6706243 B1
- US 20130272929 A1
- WO 2014135254 A1
- WO 0067805 A1